# EUROPEAN PATENT SPECIFICATION

(11) **EP 3 028 637 B1**
(45) Date of publication and mention of the grant of the patent: **20.02.2019**
(21) Application number: 14831352.1
(22) Date of filing: 19.05.2014
(51) Int. Cl.: A61B 6/04, A61B 6/14

(54) **HEAD FIXING DEVICE FOR RADIOGRAPHY IMAGING AND X-RAY IMAGING SYSTEM HAVING SAME**
KOPFFIXIERVORRICHTUNG FÜR RÖNTGENBILDGEBUNG UND RÖNTGENBILDGEBUNGSSYSTEM DAMIT
APPAREIL POUR FIXER LA TÊTE PENDANT L'IMAGERIE RADIOGRAPHIQUE ET SYSTÈME D'IMAGERIE RADIOGRAPHIQUE LE PRÉSENTANT

(30) Priority: 30.07.2013 KR 20130090005
(43) Date of publication of application: 08.06.2016
(73) Proprietor: University-Industry Cooperation Group of Kyung Hee University, Yongin-Si, Gyeonggi-Do 446-701 (KR)
(72) Inventor: KIM, Seong-Hun, Seoul 150-894 (KR); AHN, Hyo-Won, Seoul 03010 (KR); CHOI, Yong-Seok, Seoul 135-832 (KR)
(74) Representative: Schweiger, Martin
(86) International application number: PCT/KR2014/004456
(87) International publication number: WO 2015/016470

(56) References cited:
- CN-A- 102 551 772
- JP-A- 2004 000 568
- JP-A- 2006 116 051
- JP-A- 2007 307 033
- JP-A- 2008 092 990
- JP-A- 2010 214 023
- KR-A- 20100 011 301
- US-A- 4 034 748
- US-A1- 2009 307 845

## Description

### [Technical Field]

The present invention relates to a head positioning apparatus, i.e. a head fixing apparatus for holding the head of a subject in an imaging position during radiography, and more particularly, to a head fixing apparatus which allows the head to be properly positioned and stably held in place without causing deformation of facial soft tissues, and an x-ray imaging system including the same.

### [Background Art]

Photography using radiation, particularly, x-rays, is an important technique used to obtain internal images of a human body in medicine. An x-ray imaging apparatus refers to an imaging apparatus using x-rays.

Such a typical x-ray imaging apparatus using x-rays is used in various forms to photograph all or some of a human body, i.e. to obtain images related to conditions of internal structures of the human body such as internal organs or bones, a structure of teeth, a skull, and the like.

A typical x-ray imaging apparatus includes an x-ray generator generating x-rays to irradiate a subject with the x-rays, an x-ray detector detecting x-rays that are emitted from the x-ray generator and then projected onto the subject placed in a beam path, and an image output unit outputting images of the subject based on signals detected by the x-ray detector.

Here, the x-ray generator is spaced apart a predetermined distance from the x-ray detector and includes an x-ray source as a component that generates x-rays.

A typical x-ray imaging apparatus, for example, a dental computer tomographic apparatus, photographs images of teeth and/or a skull to provide x-ray images (radiographs) for dental procedures, such as orthodontic surgery or orthognathic surgery. One example of such x-ray images is disclosed in Figs. 1a and 1b of Korean Patent Publication No. 10-0904187.

In these radiographs, skin tissue (soft tissue) of a face has a relatively light color as compared with bone tissue (hard tissue) due to difference in x-ray absorption, and a contour corresponding to the shape of a skin surface is formed outside of hard tissues. Apart from dentistry, the aforementioned x-ray imaging apparatus is used in various industrial fields including fields of medicine such as plastic surgery. Particularly, the dental or plastic surgical field requires facial appearance images (facial images) of a subject in addition to the x-ray images.

Such a facial appearance image is registered with an x-ray image of a head to be used as diagnostic data for orthodontics or plastic surgery while being used in planning of surgical operations suitable for a subject or simulation for virtual plastic surgery or orthodontics.

Korean Patent Publication No. 10-2012-0096238A shows an image obtained through registration between a facial appearance image (facial image) and a cranial image (radiograph), and discloses a method of providing compensated facial images, which is capable of accurately overlapping a facial appearance image with a cranial image, and a system using the same.

In other words, Patent document 2 discloses a method of providing compensated facial images, which prefigures the facial form to be changed through a procedure planned by an operator, thereby allowing more accurate procedure planning, and a system using the same.

Documents JP-A-2006 116051 and CN-A-102 551 772 both disclose a head fixing apparatus with an occipital region support and a head holder to prevent lateral movement of the head.

An x-ray imaging apparatus for acquisition of the cranial image emits radiation to obtain x-ray images of a skull while a subject sits motionless with his/her chin on a chin rest during radiography.

In addition, the facial appearance image can be acquired by an apparatus for acquiring facial appearance images, for example, an appearance image acquisition apparatus such as a 3D scanner (face scanner). Here, the image acquisition apparatus acquires the facial image of a subject while the subject remains motionless in a predetermined imaging position after moving to a place at which the image acquisition apparatus is installed.

It should be understood that the image acquisition apparatus can acquire the facial image while the subject is held in a radiography position of the x-ray imaging apparatus before or after the cranial image is acquired by the x-ray imaging apparatus.

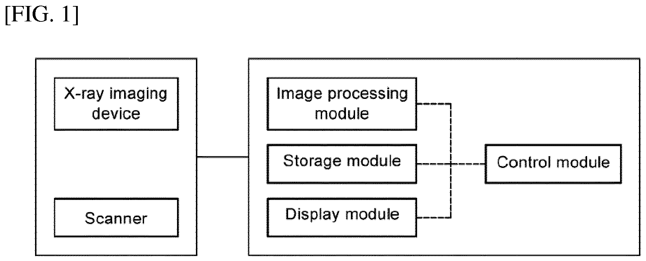

Fig. 1 is a block diagram of an exemplary image processing system of an x-ray imaging apparatus with a 3D scanner, wherein the image processing system may include: an image acquisition unit including an x-ray imaging device and a 3D face scanner (hereinafter, 'face scanner'); an image processing module acquiring the x-ray image and the facial appearance image; a storage module storing the images; a display module outputting the x-ray image and the facial appearance image on a screen; and a control module.

The x-ray imaging apparatus can acquire both the x-ray image and the facial appearance image while a subject is held in the same position with his/her chin on a chin rest without changing his/her posture or moving to another place, thereby eliminating inconvenience, which otherwise would be experienced by the subject.

When an obtained x-ray image is superimposed on a corresponding facial appearance image, the two images need to be accurately registered with each other so as to have high accuracy required for diagnostic data. As described above, the x-ray imaging apparatus including the face scanner can acquire the x-ray image and the facial appearance image while a subject is held in the same position without changing his/her posture or moving to another place, and thus can increase the degree of registration between the x-ray image and the facial appearance image while minimizing compensation of the x-ray image and/or the facial appearance image for accurate registration.

However, such a typical image processing system has the following problems in acquisition of an x-ray image and a facial appearance image and registration between the images.

If radiography is performed to obtain images of a skull, teeth, or a jawbone while a subject supports his/her chin on a chin rest, soft tissues of the chin are pressed by the chin rest, causing change of facial soft tissues, which causes deformation of a facial contour in a radiograph. Such a radiograph is difficult to use as accurate image data when it is important to obtain the contour of facial soft tissues as well as conditions of hard tissues.

In other words, since the chin is pressed by the chin rest to cause change of facial soft tissues, the degree of registration between the facial appearance image and the x-ray image is reduced, thereby making acquisition of accurate diagnostic data difficult.

In addition, in order to increase the degree of registration between the facial appearance image and the x-ray image, additional processes such as compensation of the x-ray image and/or the facial appearance image are required, thereby causing unnecessary consumption of time and manpower.

Thus, in acquisition of surface data on a facial region, i.e. facial appearance images and radiographs such as cone beam computed tomography (CBCT) images, stable fixing of a head so as to obtain images of a face (face appearance) in its natural state without change of facial soft tissues (deformation due to compression of a face against a structure such as a chin rest and deformation such as skin sagging due to gravity and depending upon the degree of muscle tension) is crucial in providing accurate information on final results of diagnosis and treatment. In addition, accurate image data is essential in exchange of procedure information between an operator and a patient and in assisting a patient in understanding the procedures.

Particularly, the dental field (orthodontics, dental surgery, prosthodontics, etc.) or the plastic surgery field requires radiographic data obtained based on the degree of radiation attenuation for a face, i.e. CBCT image data and a facial surface image for acquiring information on the color of a face and the spatial position of a face surface.

Such a facial surface image (facial image) needs to be registered with a CBCT image (head radiograph) to provide more accurate diagnostic data. Since the above two images are acquired at different times, setting the head as close to the same position and posture as possible during acquisition of the two images is important for providing more accurate diagnostic data, and also plays an important part in planning of surgical operations suitable for a subject and simulation for plastic surgery or orthodontics.

However, a radiograph in which facial soft tissues (face skin) is deformed by compression of a skin of a mental region cannot serve as accurate diagnostic data in a procedure requiring consideration of appearance.

In addition, since a contour of soft tissues in a radiograph functions as a registration reference in registration between the radiograph and a facial image, deformation of the contour of soft tissues caused by compression of a skin of a mental region makes registration between the two images difficult and thus disturbs accurate registration, thereby causing deterioration in accuracy of simulation for plastic surgery or orthodontics and the success rate of an actual procedure.

Further, dental radiography, panoramic radiography and cone beam computed tomography (CBCT) require a long radiation exposure time of 15 to 30 seconds. Here, movement of a head during panoramic radiography and CBCT causes the resultant image to have noise and artifacts and thus reduces diagnostic value of the image, or necessitates re-imaging, which increases radiation dose received by a patient, holding the head in stable position and posture to prevent movement of the head during a period of time in which a patient is exposed to radiation, i.e. during radiography, is a key factor determining successful imaging.

In the case of a disabled patient having difficulty in holding his/her head in position, for example, a patient who needs physical aids such as a wheelchair to undergo radiography, it is difficult to hold the patient in an upright posture, which makes stable radiography difficult, increases a risk of error during radiographic inspection, and causes deterioration in diagnostic value of the resultant image. Therefore, there is a need for a solution which allows imaging to be stably achieved even for a patient having difficulty in holding a posture and remaining motionless during radiography.

### [Disclosure]

### [Technical Problem]

The present invention has been conceived to overcome the aforementioned problems in the related art, and it is an object of the present invention to provide a head fixing apparatus which allows the head of a subject to be stably held in position during radiography, thereby minimizing a risk of error during radiography and providing an image having high diagnostic value, and an x-ray imaging system including the same.

It is another object of the present invention to provide a head fixing apparatus, i.e. a head positioning apparatus which supports the back of a head instead of a chin to prevent compression of facial soft tissues during x-ray imaging and acquisition of facial images, can increase accuracy of registration between an x-ray image and a facial image of a subject, and can reduce or eliminate a need for compensation of the x-ray image and the facial image, and an x-ray imaging system including the same.

### [Technical Solution]

In accordance with one aspect of the present invention, there are provided a head fixing apparatus, i.e. a head positioning apparatus which is used in an x-ray imaging system for acquisition of head images including an x-ray generator and an x-ray detector and supports the head of a subject to hold the head in an imaging position, and an x-ray imaging system using the same. Specifically, the head fixing apparatus supports the occipital region and the temporal region of a subject to position and hold the head of the subject in an imaging position.

The head fixing apparatus may be configured to prevent backward movement and lateral movement of the head. Further, the head fixing apparatus may also be configured to prevent forward movement of the head.

The head fixing apparatus includes an occipital region support (occipital support portion) supporting the back of the head to prevent backward movement of the head and a temporal region support (temporal support portion) preventing lateral movement of the head, wherein the temporal region support may be provided to the occipital region support, or may be separated from the occipital region support. In addition, the occipital region support may have a pillow shape having a predetermined curvature allowing the occipital region support to be pressed against the occipital region to support the back of the head.

The head fixing apparatus for radiography, supporting the head of a subject during radiography, i.e. a head fixing apparatus for an image acquisition apparatus including an x-ray imaging apparatus includes: an occipital region support supporting the back of the head; and a head holder movably provided to the occipital region support and tightened on the head to prevent lateral movement of the head.

The head fixing apparatus comprises a drive unit for moving the head holder, and a traction member pulling the head holder to be tightened to the head, and an actuator applying traction force to the head holder via the traction member.

### [Advantageous Effects]

A head fixing apparatus for radiography, i.e. a head fixing apparatus for holding the head of a subject in a radiography position during radiography provides the following effects.

According to the present invention, the head fixing apparatus allows the head of a subject to be stably held in position during general radiography and dental radiography for the head, thereby minimizing a risk of error during radiography and providing an image having high diagnostic value.

In addition, according to the present invention, the head fixing apparatus can minimize or prevent deformation of a facial contour caused by compression of soft tissues of a mental region, thereby allowing accurate registration between an x-ray image and a facial image and providing image data having high diagnostic value.

Further, according to the present invention, the head fixing apparatus allows registration between an x-ray image and a facial image to be easily and accurately achieved, thereby providing accurate diagnostic data and simulation data required for orthodontics or plastic surgery and allowing planning of a procedure suitable for a subject while realizing an easily comprehensible and highly recognizable image in simulation of orthodontics or plastic surgery.

### [Description of Drawings]

Fig. 1 is a block diagram of an exemplary x-ray imaging system including a face scanner.
Fig. 2 is a perspective view of a head fixing apparatus according to a first embodiment of the present invention.
Fig. 3 is a schematic perspective view of the head fixing apparatus shown in Fig. 2, with the head of a subject supported thereby.
Fig. 4 is a rear view of the head fixing apparatus shown in Fig. 2.
Fig. 5 is a plan view of the head fixing apparatus shown in Fig. 2.
Fig. 6a is a view showing operation of a head fixing apparatus according to one embodiment of the present invention.
Fig. 6b is a cross-sectional view taken along line I-I oh Fig. 6a.
Fig. 7a is a view showing operation of a head fixing apparatus according to another embodiment of the present invention.
Fig. 7b is a cross-sectional view taken along line II-II of Fig. 7a.
Fig. 8a is a view showing operation of the head fixing apparatus according to a further embodiment of the present invention.
Fig. 9 is a plan view showing operation of the head fixing apparatus shown in Fig. 8.
Fig. 10 is a view showing operation of a head fixing apparatus according to yet another embodiment of the present invention.
Fig. 11 is a schematic view of a head fixing apparatus according to another embodiment of the present invention.
Fig. 12 is a view showing the head fixing apparatus shown in Fig. 11.
Fig. 13 is a side view of the head fixing apparatus shown in Fig. 11.
Fig. 14 is a perspective view of the head fixing apparatus shown in Fig. 11.
Fig. 15 is a perspective view of an exemplary image acquisition apparatus i.e. an x-ray imaging system including the head fixing apparatus according to the present invention.
Fig. 16 is a front view of the head fixing apparatus shown in Fig. 11, with a subject placed thereon.
Fig. 17 is a schematic front view of a head fixing apparatus according to a further embodiment of the present invention.
Fig. 18 is a view of a head fixing apparatus according to yet another embodiment of the present invention.

### [Best Mode]

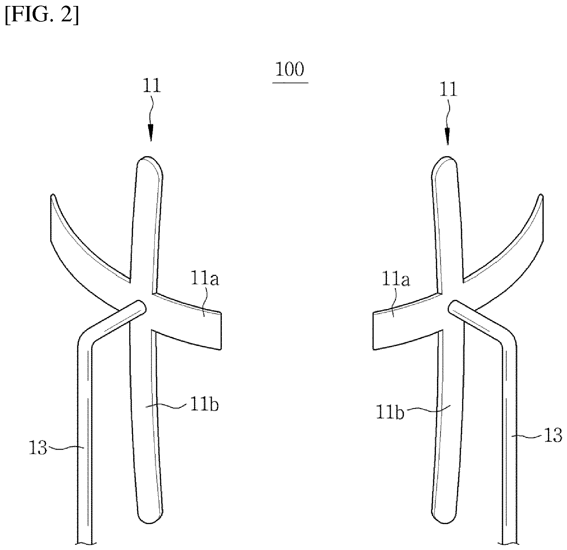

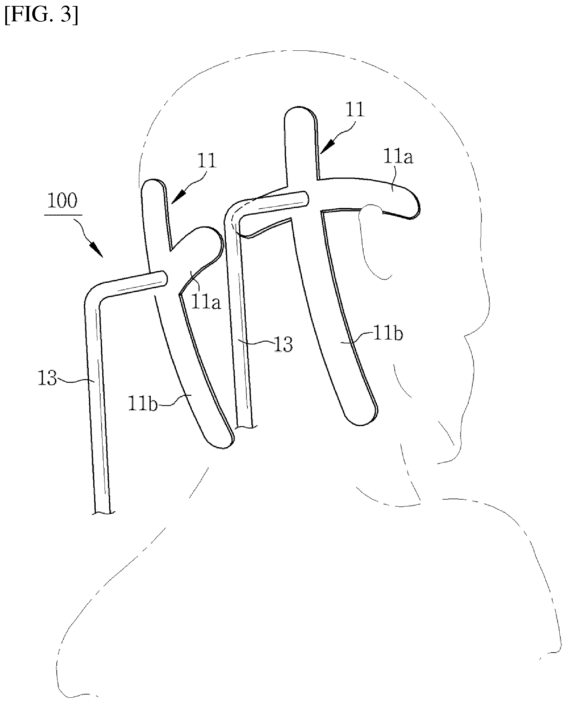

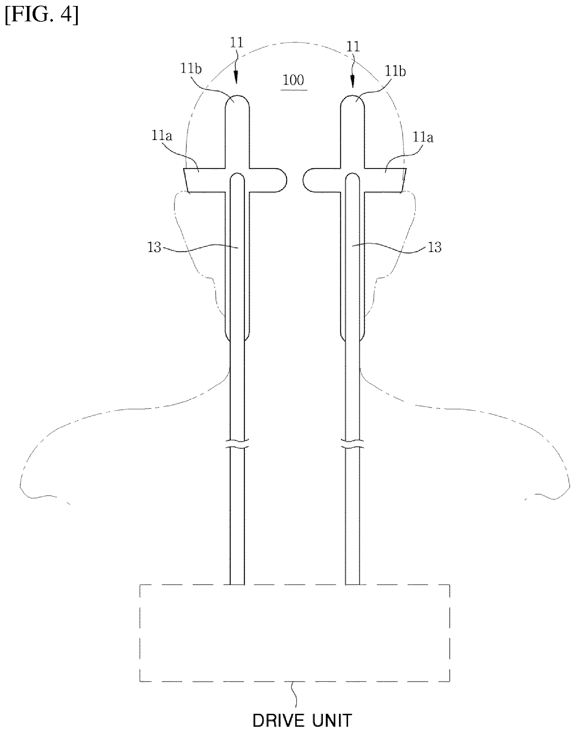

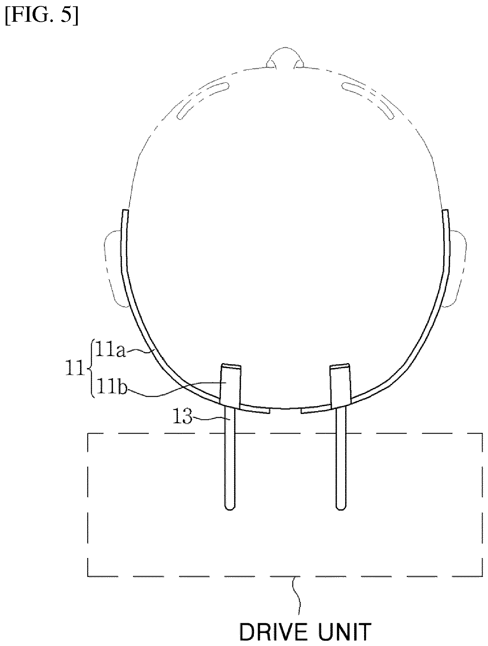

Hereinafter, embodiments of the present invention will be described in detail with reference to Figs. 2 to 10. In descriptions of embodiments, like components will be referred to by like names and like reference numerals, and additional descriptions thereof may be omitted.

It should be understood that embodiments of the invention may be used in medicine such as plastic surgery as well as for dental purpose. In addition, an x-ray imaging system, i.e. a head image acquisition system using a head fixing apparatus according to the present invention may further include a device for acquisition of a facial image, particularly, a 3D image.

First, one embodiment of the invention will be described with reference to Figs. 2 to 5.

Referring to Figs. 2 to 5, a head fixing apparatus 100, i.e. a head positioning apparatus according to one embodiment of the invention is used in an x-ray imaging apparatus for acquisition of a head image, i.e. a head image acquisition system including an x-ray generator and an x-ray detector, and supports the head of a subject to hold the head in an imaging position.

The head positioning apparatus 100 supports the occipital region and the temporal region to prevent the head of a subject from moving backward and from side to side. Further, the head holding device may also be configured to prevent the head of a subject from moving forward.

In other words, the head fixing apparatus 100 supports the occipital region and the temporal region to hold the head in an imaging position such that imaging can be achieved without change of facial soft tissues (face skin) during x-ray imaging and face scanning. According to embodiments of the invention, the head fixing apparatus may be configured not to hide the front of a subject.

The head fixing apparatus 100 has a head support 11 supporting the back of a head to prevent the head from moving backward and supporting lateral regions of the head, particularly, regions above ears to prevent the head from moving from side to side. It is desirable in terms of preventing compression of facial soft tissues (face skin) that the head fixing apparatus 100 be configured to support a scalp region (region with hairs).

The head support 11 is symmetrically placed on both sides of the back of the head, and includes an occipital region support for supporting the back of the head (occipital region) and a temporal region support for supporting the temporal region, wherein the temporal region support is provided to the occipital region. Specifically, the temporal region support and the occipital region support are integrally formed with each other and behave integrally.

Specifically, the head support 11 includes a bar-type horizontal support portion 11a transversely pressed against the back of the head to horizontally support the back of the head, and a bar-type vertical support portion 11b longitudinally pressed against the back of the head to vertically support the back of the head. In addition, the horizontal support portion 11a and the vertical support portion 11b are integrally formed with each other, and the head support 11 functions as both the occipital region support and the temporal region support. In other words, in this embodiment, the horizontal support portion 11a extends to the temporal region to support a lateral side of the head, whereby the head support 11 has both a function of supporting the occipital region and a function of supporting the temporal region.

However, it should be understood that the horizontal support portion 11a may only support the back of the head, and a temporal region support for preventing lateral movement of the head may be separately provided. The structure wherein the temporal region support and the occipital region support are separated from each other will be described in another embodiment set forth below.

Further, the head positioning apparatus, i.e. the head fixing apparatus 100, is adjustable in width and may also be movable up or down. The head fixing apparatus 100 includes a drive unit for width adjustment and/or lifting, wherein the drive unit includes a width adjustment unit for adjusting width of the head support 11, particularly, width of portions supporting the temporal regions (temporal support portions), and a lifting unit for lifting of the head support 11, particularly, for adjusting height of a portion supporting the occipital region (occipital support portion). The head fixing apparatus has a structure wherein the temporal support portion (temporal region support) is provided to the occipital support portion (occipital region support), particularly, a structure wherein the occipital support portion is integrally formed with the temporal support portion. Thus, the width of the temporal support portions is adjusted by adjusting the width of a pair of the head supports 11 placed on both sides of the back of the head, and the heights of the occipital and temporal support portions are adjusted by moving the head support 11 up or down.

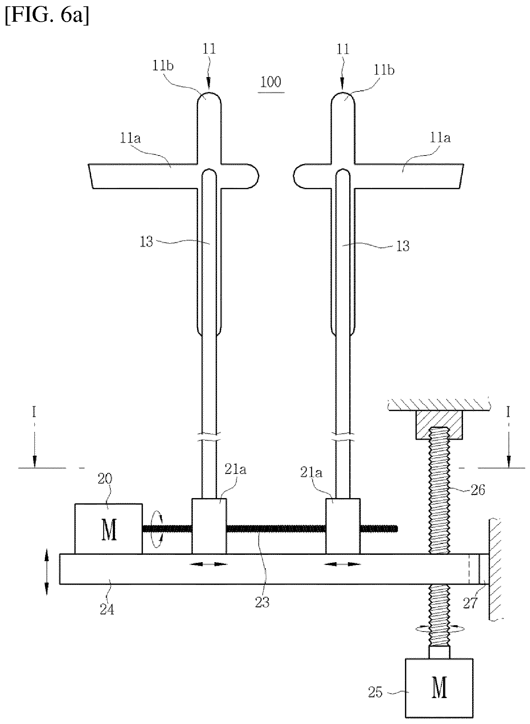

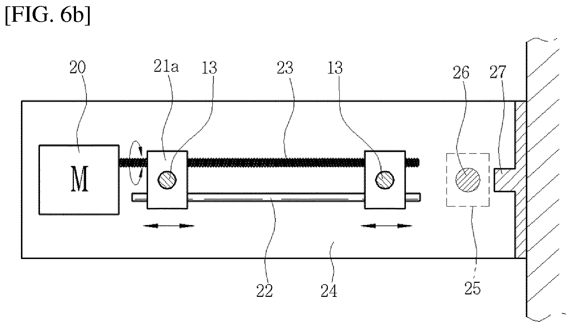

Referring to Figs. 6a and 6b, the width adjustment unit includes right and left linear drive blocks 21a, 21b to which the right and left occipital region supports 11 are fixed, respectively, a first guide rail 22 guiding linear movement of the right and left linear drive blocks 21a, 21b, a first lead screw 23 rotatably mounted through the right and left linear drive blocks 21a, 21b, and a drive motor 20 transmitting torque to the first lead screw 23. As the first lead screw 23 is rotated, the width between the pair of the head supports 11 becomes narrower or wider by the first lead screw 23.

Here, since the first lead screw 23 is threaded in opposite directions at opposite sides of an axial center thereof, the right and left linear drive blocks 21a, 21b are moved in opposite directions when the first lead screw is rotated in one direction.

Alternatively, the first lead screw 23 may be rotated manually rather than being automatically rotated by the drive motor 20. For this, the width adjustment unit may be provided with a handle for turning the first lead screw 23.

The lifting unit includes a mount plate 24 on which components of the width adjustment unit, particularly, the right and left linear drive blocks 21a, 21b, the first guide rail 22, and the first lead screw 23 are mounted; a second lead screw 26 vertically mounted through the mount plate 24 in a rotatable manner; and a second guide rail 27 guiding linear movement of the mount plate 24. Rotation of the second lead screw 26 causes the mount plate 24 to be moved up or down.

Alternatively, the second lead screw 26 may be activated manually rather than being activated by the drive motor 25. For this, the lifting unit may be connected to a turning handle (not shown).

Further, the horizontal support portion 11a or the vertical support portion 11b may be provided with an ear road (not shown) which is placed inside the ear (in the external acoustic pore) at an end thereof to aid positioning of the head. The ear road is well known in the art, and detailed descriptions thereof will be omitted herein.

The pair of the head supports 11 is provided to support arms 13 to be supported by the support arms 13 and are connected to the right and left linear drive blocks 21a, 21b via the respective support arms 13, respectively. It should be understood that the vertical support portions 11b may be directly connected to the right and left linear drive blocks 21a, 21b, and, in this case, the vertical support portions have a shape and structure which do not interfere with the neck or the back.

Further, each of the head support 11 and the support arm 13 may be formed of an elastic material. Specifically, each of the head support 11 and the support arm 13 may be formed of an elastic hard material having high x-ray transmittance, such as acrylic resins, carbon fiber-reinforced plastics, or polycarbonate.

Next, advantageous effects of the head fixing apparatus 11 according to this embodiment will be described.

According to this embodiment, in x-ray imaging and/or face scanning, the head fixing apparatus allows x-ray images and facial appearance images to be obtained without change of a posture while the occipital and temporal regions of a subject, instead of the chin, are supported. Here, the head fixing apparatus 11 does not hide the front of the face. Further, the pair of head supports 11 is adjustable in height and width depending upon physical conditions of a subject, for example, stature, sitting height, and size (length or width) of a head.

Specifically, since rotation of the second lead screw 26 by the drive motor 25 causes the mount plate 24 to be moved up or down, the head support 11 is moved up or down depending on the rotational direction of the drive motor 25.

In addition, rotation of the first lead screw 23 by a separate drive motor 20 causes the right and left linear drive blocks 21a, 21b to become close to or away from each other, whereby width between the head supports 11 can be adjusted.

The head fixing apparatus according to this embodiment can prevent compression of facial soft tissues during acquisition of a head image through x-ray imaging and face scanning, allows easy registration between an x-ray image and a facial appearance image, can provide more accurate diagnostic data to an operator and a patient, and is thus very effective in a virtual procedure simulation.

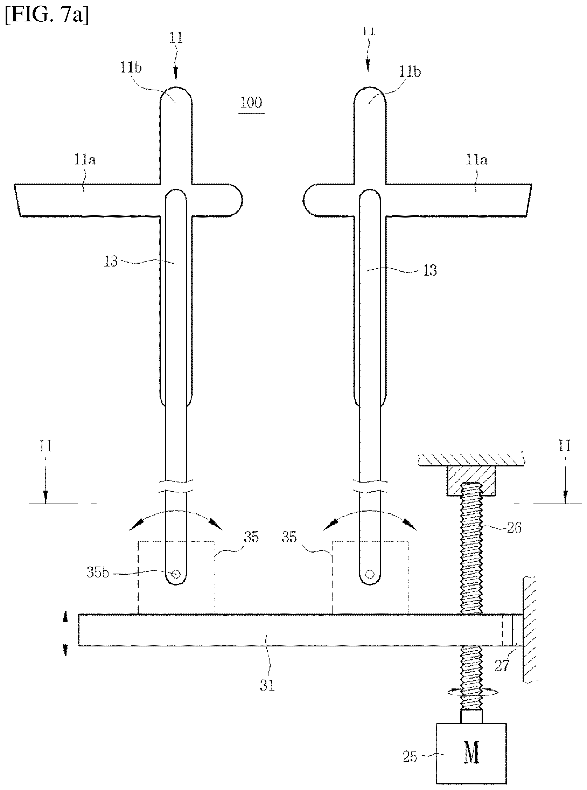

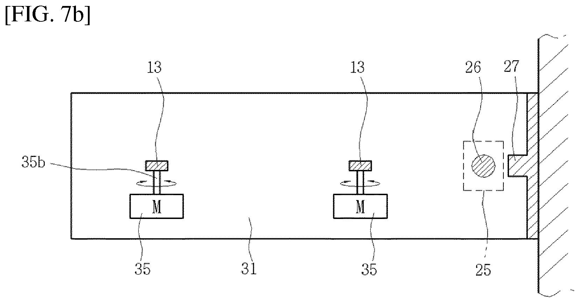

Next, a head fixing apparatus will be described with reference to Figs. 7a to 7b. Referring to Figs. 7a to 7b, a head fixing apparatus 100 includes a head support 11, a width adjustment unit, and a lifting unit. The width adjustment unit and the lifting unit are the same in functionality as those of the above embodiment, but different in terms of structure and operation than those of the above embodiment. To the head supports, the same descriptions as in the above embodiment can be applied.

In this embodiment, a pair of head supports 11 becomes wider or narrower in width between upper end portions thereof by being rotated. Specifically, the width adjustment unit according to this embodiment includes a drive motor 35 rotating the head support 11 clockwise or counterclockwise and a mount plate 31 on which the drive motor 35 is mounted. Here, the drive motor 35 is placed on each side of the mount plate.

In this embodiment, although the head support 11 is indirectly coupled to the drive motor 35 via a support arm 13, it should be understood that the drive motor 35 may be directly coupled to a lower end of a vertical support portion 11b without the support arm 13.

In addition, the lifting unit includes a lead screw 26 vertically mounted through the mount plate 31 constituting the width adjustment unit in a rotatable manner, and a guide rail 27 guiding linear movement of the mount plate 31, wherein the mount plate 31 is moved up or down depending upon the rotational direction of the lead screw 26.

Here, the lead screw 26 may be automatically activated by the drive motor 25 or may be activated manually, as described in the above embodiment.

As the lead screw 26 is rotated manually or by the drive motor 25 for imaging of a head, the head support 11 is moved up or down along with the mount plate 31 moved in connection with the lead screw 26.

Once adjustment of height of the head support 11 is completed, the width between the vertical support portions 11b is adjusted to fit head size (width) of a subject.

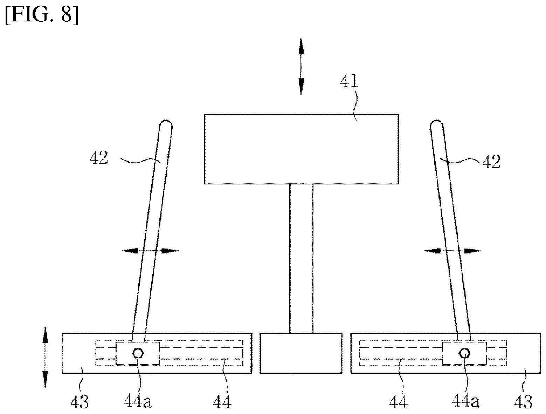

Specifically, when the drive motor 35 for width adjustment is driven, the head support 11 is also rotated according to the rotational direction of the drive motor 35 for width adjustment, as indicated by arrows in Fig. 8a, which causes width between upper ends of the head supports 11 to be reduced or increased.

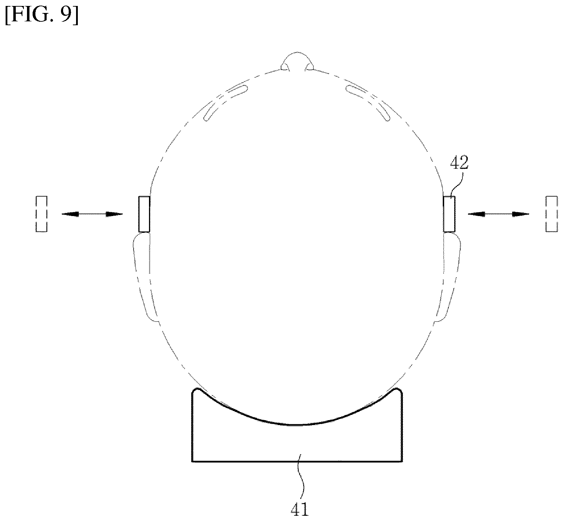

Next, referring to Figs. 8 and 9, a head fixing apparatus may include an occipital support portion 41 (occipital region support) and temporal support portions 42 (temporal region supports), wherein the occipital region support 41 has a pillow shape. Although the temporal support portion is provided to the occipital support portion in the above embodiments, the occipital support portion 41 is separated from the temporal support portion 42.

Specifically, the head fixing apparatus includes the occipital region support 41 supporting the back of the head to prevent backward movement of the head, and the temporal region supports 42 separated from the occipital region support 41 and supporting the temporal regions, specifically lateral regions above ears (scalp regions) to prevent lateral movement of the head.

In this embodiment, the occipital region support 41 has a pillow shape having a predetermined curvature that allows the occipital region support to be pressed against the occipital region to support the back of the head. In addition, the head fixing apparatus further includes a lifting unit for raising and lowering the occipital region support 41. The lifting unit is configured to move the occipital region support 41 up or down, and is operable using various means such as lead screws described in the above embodiments, solenoids, pneumatic actuators, and hydraulic actuators.

In addition, the temporal region support 42 is provided to a mount plate 43 and, in this embodiment, is mounted on the mount plate 43 in a horizontally movable manner. The temporal region support 42 is provided at a lower end thereof with a fixing member 44a for fixing the position of the temporal region support 42.

The fixing member 44a is a screw type member. When the fixing member 44a is loosened, the temporal region support 42 is separated to be movable horizontally, whereas, when the fixing member 44a is tightened, the temporal region support 42 is fixed in position. However, means for fixing the temporal region support 42 are not limited thereto.

In addition, in this embodiment, the temporal region support 42 may be provided with an ear road to aid positioning of the head. Further, the occipital region support 41 and the temporal region support 42 may be formed of the same material as that described in the above embodiments.

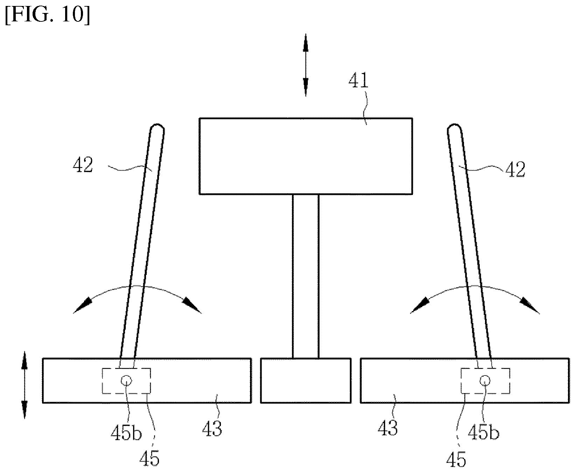

The temporal region supports 42 may be rotated to retract or spread to be adjusted in width therebetween. Fig. 10 is a view of a head fixing apparatus according to yet another embodiment of the invention wherein the temporal region support 42 is a lever type support rotatable about a lower end thereof.

Referring to Fig. 10, in this embodiment, each of the temporal region supports 42 is provided to the mount plate 43. In addition, each of the temporal region supports 42 is mounted to be rotatable about a motor shaft 45b by a drive motor 45 for width adjustment such that upper ends of the temporal region supports 42 spread or retract to be adjusted in width therebetween.

According to the above embodiments, the head fixing apparatus allows a head image, i.e. an x-ray image and/or a facial image, to be obtained without hiding the front of the face of a subject while preventing compression, i.e. deformation of face skin (facial soft tissues).

The width adjustment unit, the lifting unit, and the head support described in the above embodiments may be altered in structure and shape in various ways, and the temporal support portion may be connected to or separated from the occipital support portion. Further, the head fixing apparatus according to the present invention may be used not only in an x-ray apparatus for providing radiographs (radiography system) but also in an apparatus in which an apparatus having a function to obtain radiographs, for example, the x-ray apparatus and a facial image acquisition device are integrated as a single system, i.e. a complex apparatus. In the latter case, it is possible to obtain both a facial image and a head radiograph in the same posture and position. In addition, it should be understood that the head fixing device according to the invention may be used in radiographic apparatuses for various applications such as plastic surgery as well as for dental purposes.

The head fixing apparatus may be provided to a upper structure, for example, a frame of an imaging apparatus for supporting an x-ray generator and an x-ray detector, or to a lower structure, for example, the back of a chair or a bed.

### [Mode for invention]

A head fixing apparatus according to embodiments to be described below (hereinafter, "head fixing apparatus") serves to support the head of a subject during radiography, and includes an occipital region support for supporting the back of the head and a head holder provided to the occipital region support to prevent lateral movement of the head, wherein the head holder serves to support the temporal regions.

Specifically, the head holder is movably provided to the occipital region support and is tightened on the head to prevent lateral movement of the head. Thus, the head fixing apparatus may be used not only in radiography for acquisition of a head image but also in photography for acquisition of a facial image.

The head holder includes: a left support movably provided to the occipital region support to press a left side of the head; and a right support movably provided to the occipital region support to press a right side of the head. In other words, the head holder may be divided on both sides of the occipital region support.

Alternatively, the head holder may include a head fixing band wrapped around the temporal regions and the frontal region of the head to be tightened on the head and pressing the back of the head against the occipital region support.

The occipital region support may be movable up or down for height adjustment.

In addition, the head holder may be moved automatically or manually to be tightened on the head. The head fixing apparatus according to the present invention may further include a drive unit moving the head holder to be tightened on the head.

The drive unit may include: a traction member pulling the head holder to be tightened on the head, and an actuator applying traction force to the head holder via the traction member.

The occipital region support is supported by a backrest, and the traction member is placed in a traction path formed in the occipital region support and the backrest. In addition, the occipital region support may be connected to the backrest via a neck support supporting the back of the head.

The neck support has a forwardly convex shape such that a cervical spine is curved in the form of the letter "C".

The neck support may include: a support frame, and a cushion provided to the support frame and inflated according to the amount of a fluid supplied thereto to support the back of the neck such that a cervical spine is curved in the form of the letter "C".

The backrest may be mounted on a backrest support (backrest stand), and the backrest may be provided with a backrest fastening belt fastening the backrest to the backrest support. In addition, the backrest may be provided with a subject fastening belt fastening the body of a subject to the backrest.

The head fixing apparatus may further include a seat supporting hips to allow a subject to sit thereon.

The head holder may be a bar made of an elastic material or a band made of a flexible material, and the occipital region support may be made of an acrylic resin, without being limited thereto.

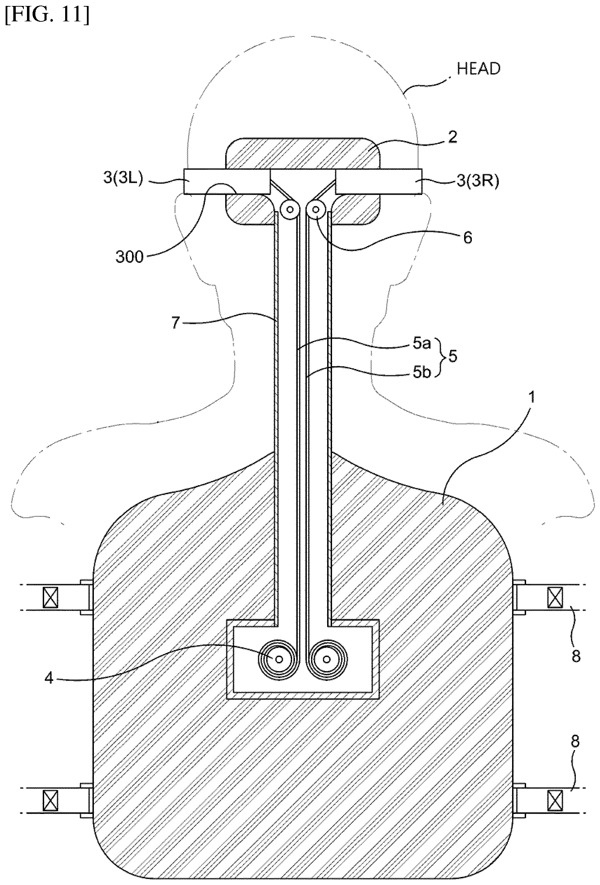

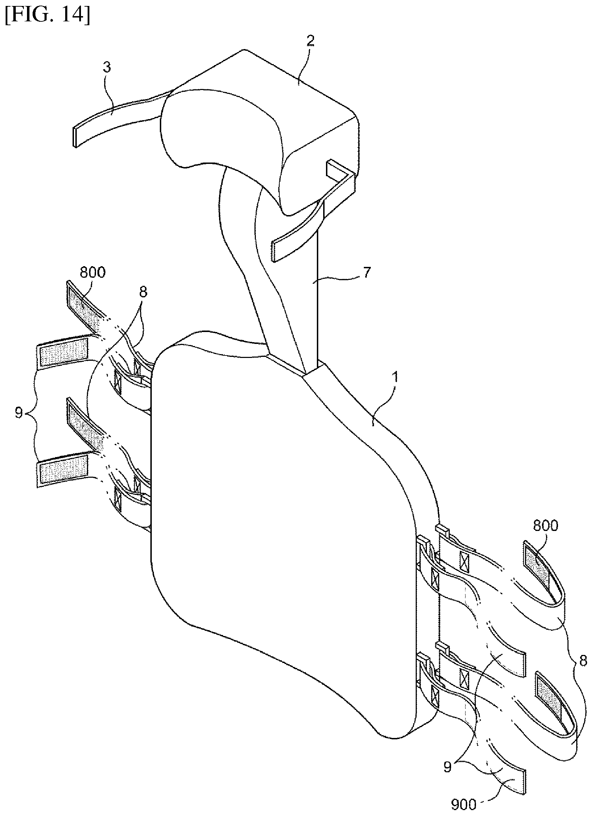

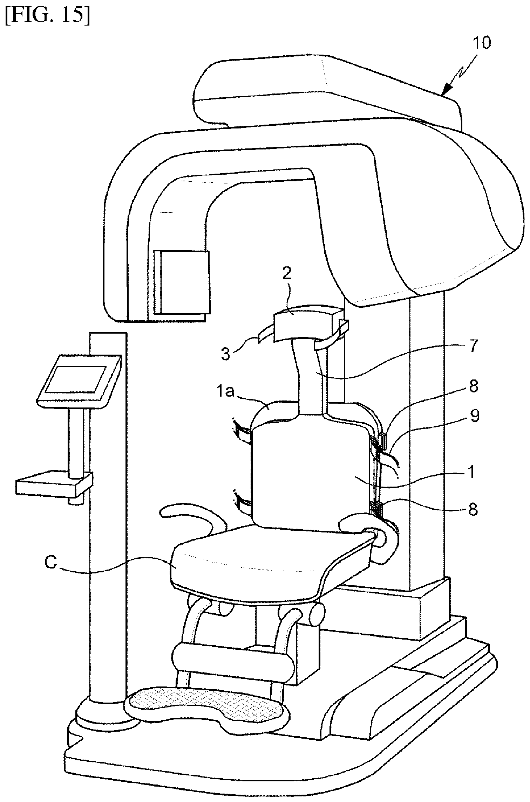

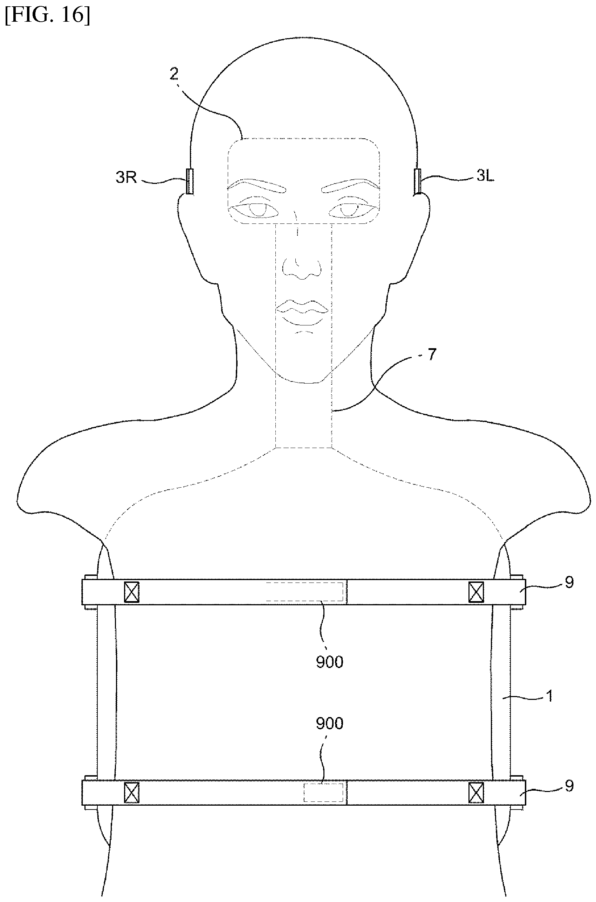

Hereinafter, additional embodiments of a head fixing apparatus will be described with reference to Figs 11 to 16. It should be noted that like components will be denoted by like reference numerals.

Referring to Figs. 11 to 16, a head fixing apparatus includes an occipital region support 2 and a head holder 3. Here, the occipital region support 2 is a structure (head rest) supporting the back of the head, i.e. the occipital region.

The head holder 3 is configured to be pressed against the head to fix the head on the occipital region support 2, specifically to be tightened on the head to fix the head on the occipital region support 2, thereby preventing lateral movement of the head.

The occipital region support 2 may be suspended from an upper structure, for example, a body 10 supporting an x-ray generator and an x-ray detector, or may be supported by a lower structure, for example, a backrest as described below. Although, in this embodiment, the occipital region support 2 is supported by the backrest, and the head holder 3 is movably provided to the occipital region support 2, the present invention is not limited thereto.

The head holder is moved by power (driving force) of a drive unit to be pressed against the head, thereby restricting movement of the head and maintaining the position and posture of the head of a subject during radiography.

Specifically, the head fixing apparatus includes a backrest 1 supporting the back of a subject, the occipital region support 2 connected to the backrest 1, the head holder 3 movably provided to the occipital region support 2 and pressed against the head of a subject, particularly, lateral sides of the head to fix the head, and a drive unit providing driving force to the head holder 3 such that the head holder 3 is moved to be pressed against the head.

The drive unit includes a traction member 5 pulling the head holder 3 and an actuator applying traction force to the head holder 3 via the traction member 5, for example, a motor.

The drive unit includes a motor for providing driving force (not shown, actuator), a take-up roll 4 rotated by the motor, and the traction member 5 connected to the head holder 3 and wound on the take-up roll 4 to pull the head holder 3. As the traction member 5 pulls the head holder 3 while being wound on the take-up roll 4, the head holder 3 is pressed against the head to be tightened on the head, whereby the head of a subject is fixed to a front surface of the occipital region support 2.

In addition, the traction member 5 vertically extends from the occipital region support 2 to the backrest 1, and the drive unit may further include a component supporting the traction member 5 to redirect the traction force, i.e. a guide roller 6 converting vertical traction force applied to the traction member 5 into horizontal traction force.

The traction member 5 is installed in a traction path formed in the occipital region support 2 and the backrest 1 and transmits driving force of the actuator, i.e. the motor, to the head holder 3. The motor and the take-up roll 4 may be mounted within the backrest 1, without being limited thereto. Alternatively, the motor and the take-up roll may be installed outside the backrest 1.

The head holder 3 has elasticity and is composed of portions placed in guide holes 300 formed on both sides of the occipital region support 2 and portions extending forward of the occipital region support 2.

Specifically, the traction member 5 includes a left traction portion 5a connected to a left portion of the head holder 3 and a right traction portion 5b connected to a right portion of the head holder 3.

Here, the left portion of the head holder 3 is mounted on and supported by a left portion (left guide hole) of the occipital region support 2, and the right portion of the head holder 3 is mounted on and supported by a right portion (right guide hole) of the occipital region support 2.

The head holder 3 includes a left support 3L movably provided to the occipital region support 2 to press a left side of the head and a right support 3R movably provided to the occipital region support 2 to press a right side of the head.

A rear end of the left support 3L and a rear end of the right support 3R are placed in the guide holes 300, respectively, to be movable axially (in a longitudinal direction of the guide hole), wherein the guide holes are formed on both sides of the occipital region support 2.

Each of a portion of the left support 3L forwardly extending from the rear end thereof to press the left side of the head and a portion of the right support 3R forwardly extending from the rear end thereof to press the right side of the head has a curved concave shape at an inner surface thereof to be pressed against the head along a surface of each of the temporal regions.

The head holder 3, i.e. the left support 3L and the right support 3R, may be a bar formed of an elastic material.

In addition, the head fixing apparatus includes a connector 7 between the backrest 1 and the occipital region support 2, wherein the connector connects the backrest 1 to the occipital region support 2 to support the occipital region support 2. In this embodiment, the connector 7 serves as a neck support supporting the back of a neck (cervical region).

The connector, i.e. the neck support 7 may have a path of the traction member 5 therein. In this embodiment, the neck support 7 is hollow, and thus an internal space (hollowness) of the neck support 7 forms a path of the traction member 5.

Preferably, the neck support 7 has a forwardly concave shape such that the cervical spine is curved in the form of the letter "C". In other words, it is desirable that the neck support 7 have a curved shape such that the side of the neck support facing the neck (cervical region) of a subject is pressed against the neck along the normal curve of the cervical spine and also the spinal column when a normal subject holds a pose. The neck support 7 may be configured to support at least some of the spinal column, for example, the upper spine in addition to the cervical spine.

Preferably, the neck support 7 is partially formed of an elastic material. For example, the backrest 1 and the neck support 7 may have a structure wherein a cushion member such as a cushion filled with sponge, cotton wool, or a fluid to provide shock absorption and support is coupled to an acrylic support frame, without being limited thereto.

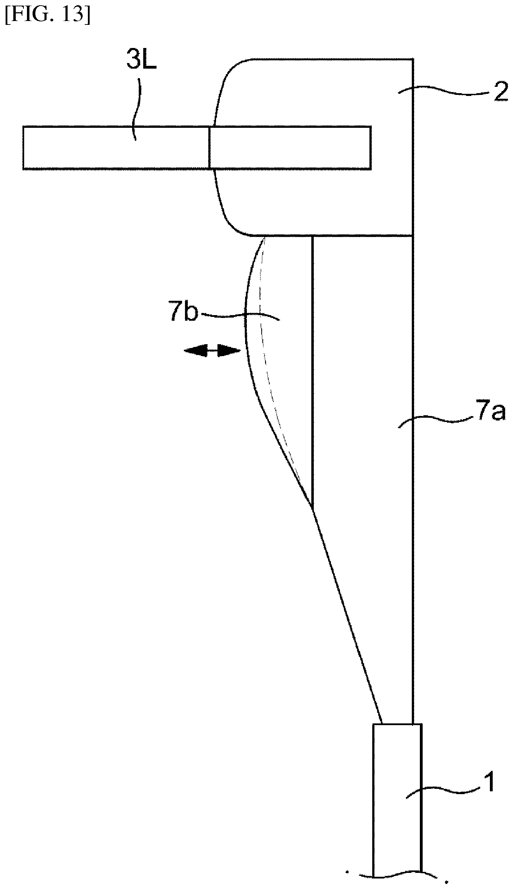

Referring to Fig. 13, the neck support 7 may include a support frame 7a and a cushion 7b provided to the support frame 7a and inflated by being filled with a fluid.

The cushion 7b is configured to be inflated according to the amount of a fluid when being filled with a fluid such as air, and is inflated from the volume indicated by the dotted line to the volume indicated by the solid line in Fig. 13 to fit the physique of a subject such that the cervical spine is curved in the form of the letter "C", thereby supporting the back of the neck. Although not shown, the cushion 7b is connected to a fluid supply pipe for filling the cushion with a fluid. Since air-filling techniques using the fluid supply pipe, for example, air supply pipe are widely known per se, detailed descriptions thereof will be omitted.

The head fixing apparatus according to embodiments of the invention may further include a seat C (hip support) that supports hips to allow a subject to sit thereon. In other words, although the head fixing apparatus may fix the head of a subject while the subject stands, the head fixing apparatus may also fix the head of a subject while the subject sits down, i.e. takes a seat or lies.

In addition, the seat, i.e. the hip support, may be configured to be movable. For example, the seat may be configured to be movable up or down and/or movable from side to side. When the seat is configured to be movable up or down, the level of the head of a subject can be adjusted to fit an imaging position. Since the moving mechanism of the seat is widely known in the fields of various seats, for example, automobile seats, detailed descriptions thereof will be omitted.

The backrest 1 may be removably mounted on the backrest support for supporting the backrest 1, for example, the back 1a of a typical chair. For this, the backrest 1 may be provided with a band type backrest fastening belt 8 for fastening the backrest 1 to the backrest support 1a. Accordingly, the backrest 1 may be attached to/detached from the backrest support, and thus can be used in a chair of a typical x-ray apparatus.

In other words, the backrest 1 of the head fixing apparatus may be securely bound to the back 1a of a chair (chair with a back and a seat) using the backrest fastening belt 8. Here, the backrest fastening belt 8 may include a pair of the backrest fastening belts provided at lateral sides of each of upper and lower halves of the backrest 1, without being limited thereto.

In addition, the backrest 1 may be provided with a subject fastening belt 9 for fastening the body of a subject to the backrest 1. Although, in this embodiment, two pairs of the subject fastening belts 9 are provided to bind both the chest and the abdomen of a subject, the present invention is not limited thereto.

In other words, the subject fastening belt 9 is forwardly moved to be wrapped around the body of a subject, and provides clamping force such that the subject can be tightly fastened to the backrest 1.

Although, in order to maintain clamping force of the backrest fastening belt 8 and the subject fastening belt 9, the belts are provided with Velcro-type fasteners 800, 900, respectively, it should be understood that a well-known mechanism for providing clamping force used in automobile or airplane seat belts, or a typical mechanism for providing clamping force to waist belts may be employed.

Next, advantageous effects of the head fixing apparatus will be described.

The head fixing apparatus supports the occipital region and temporal regions of a subject to stably place and hold the head of the subject in an imaging position during acquisition of radiographs and also facial images such that imaging can be stably achieved without changes of facial soft tissues (face skin), particularly, soft tissues near a facial midline running from the tip of the chin to the glabella.

Here, the head holder 3 is preferably configured to support a scalp region (soft tissue region with hairs), i.e. not to press a face skin region (facial soft tissue region). In this embodiment, the head holder includes portions separately placed on both sides of the head to be symmetric to each other.

The occipital region support 2 is pressed against the back of the head to support of the back of the head (occipital region), thereby preventing the head from tilting back.

Preferably, each of the head holder 3 and the neck support 7 is formed of an elastic material so as to effectively support body parts in contact therewith, such as the head, the cervical spine, and the like while providing shock absorption.

Specifically, each of the head holder 3 and the neck support 7 is formed of an elastic hard material having high x-ray transmittance, such as acrylic resins, carbon fiber-reinforced plastics, or polycarbonate to exhibit a certain level of solidity and ergonomic suitability, and the traction member 5 may be in the form of a flexible belt or wire made of synthetic resin (nylon, polyethylene, fluorocarbon, etc.).

Thus, the head fixing apparatus allows the head of a subject to be fixed to the occipital region support 2 through clamping action, thereby allowing radiographs to be obtained without change in head position or posture. Specifically, once the head of a subject is placed in front of the occipital region support, a motor provided to the backrest 1 is driven to cause the take-up roll 4 to be rotated. As a result, as the traction member 5 is wound onto the take-up roll 4, the head holder 3 connected to the traction member 5 is moved in a direction of being pressed against lateral sides of the head of a subject.

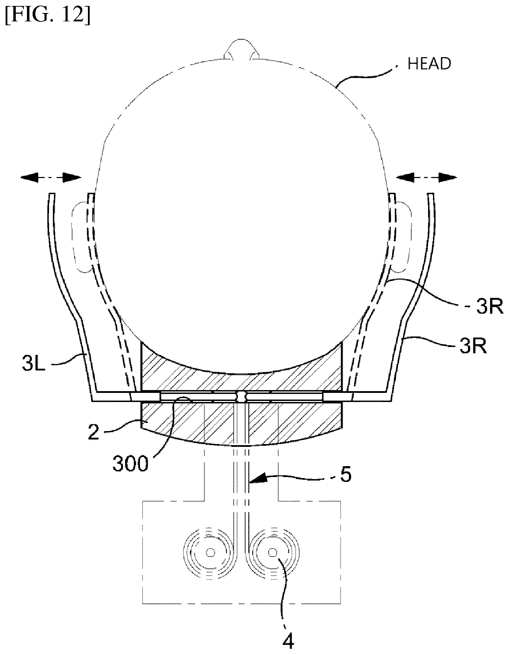

In other words, as shown in Fig. 12, the head holder 3 is moved from the position indicated by the solid line to the position indicated by the dotted line by traction force of the traction member 5 applied by the actuator, thereby being pressed against the temporal regions of a subject, i.e. lateral sides of the head.

As such, when the head holder 3 is pressed against the lateral sides of the head of a subject, the head can be naturally hold in position, thereby securing stability during radiography.

In addition, the head fixing apparatus may be mounted on a typical chair via the backrest fastening belt 8, and allows an upper body of a subject to be tightly fastened to the backrest 1 using the subject fastening belt 9 when the subject has difficulty maintaining body balance for a long time because the subject is elderly, infirm, or disabled.

Further, since the neck support 7 supporting body parts from the back to the neck is pressed against the body parts along the curve of the cervical spine and is made of an elastic material, the head fixing apparatus is ergonomically designed, thereby providing tight fit to the human body and stable support properties.

It is possible to reduce or prevent compression of face skin including skin of the mental region during acquisition of radiographs and also facial images, thereby providing accurate diagnostic data.

In addition, the head fixing apparatus allows a radiograph and a facial image to be obtained in the same position and posture. Thus, in registration between the images, it is possible to minimize or eliminate a need for a separate image compensation process for registration between the radiograph (x-ray image) and the facial image (face appearance image) and to plan a surgical procedure suitable for a subject while allowing simulation of orthodontics or plastic surgery close to actual procedure results.

The occipital region support 2 may be configured to be movable up or down for height adjustment. In other words, the occipital region support 2 may be moved up or down together with the backrest 1, or the occipital region support 2 may be moved up or down with the backrest 1 held in place as in an embodiment to be described below.

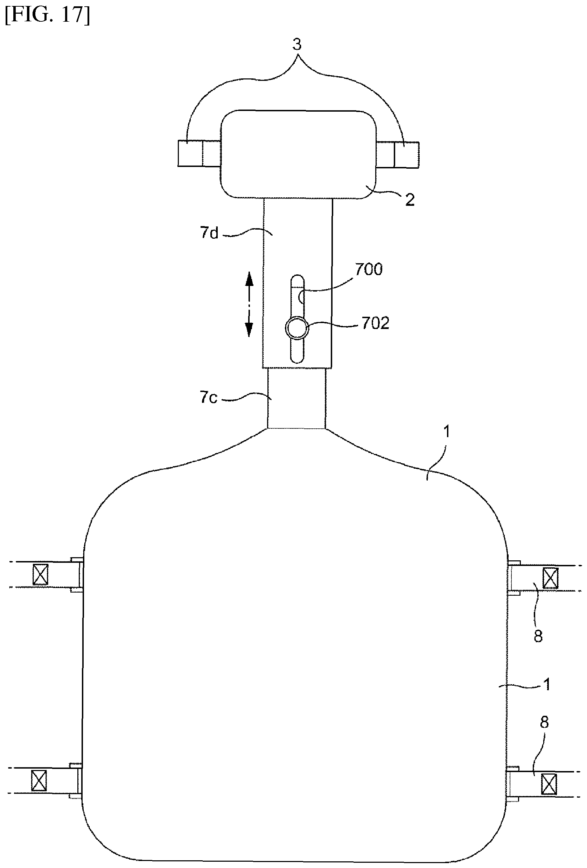

Next, one embodiment of the lifting mechanism of the occipital region support will be described in detail. Referring to Fig. 17, the neck support 7 may be separated into two portions such that the height of the occipital region support 2 can be properly adjusted depending upon the physique of a subject.

In other words, the neck support may have a telescopic structure. Specifically, the neck support 7 includes a lower part 7c mounted on the backrest 1, an upper part 7d movably provided to the lower part 7c, and a fastener for fixing the upper part 7d to the lower part 7c.

More specifically, the upper part 7d is removably provided to the lower part 7c and configured to slide vertically to be adjusted in level depending upon the height of a subject.

The fastener may include a long guide hole 700 formed in the upper part 7d in a longitudinal direction and a fastening screw coupled to a screw hole of the lower part 7c through the long guide hole 700 to fix the upper part 7d to the lower part 7c. It should be understood that the configuration of the fastener is not limited thereto.

For example, the neck support may have a structure wherein multiple upper parts are inserted into the lower part to be telescopically drawn. By way of another example, the fastener may have a structure wherein at least one pin-insertion hole is formed in any one of the lower part and the upper part, and a plurality of pin-insertion holes is formed in the other part to be vertically spaced apart from one another, such that the occipital region support 2 can be adjusted in level depending upon relative positions between pin-insertion holes of the lower and upper parts. It should be understood that the fastener for adjusting the level of the neck support separated into the upper and lower parts may be configured in various ways known in the art.

The head holder 3 may be provided with an ear road (not shown) which extends from the head holder 3 and is placed inside the ear (in the external acoustic pore) at an end thereof to aid positioning of the head.

The ear road for aiding positioning of the head is widely known, and may be a structure branched from the head holder 3, i.e. a branched structure, in this embodiment.

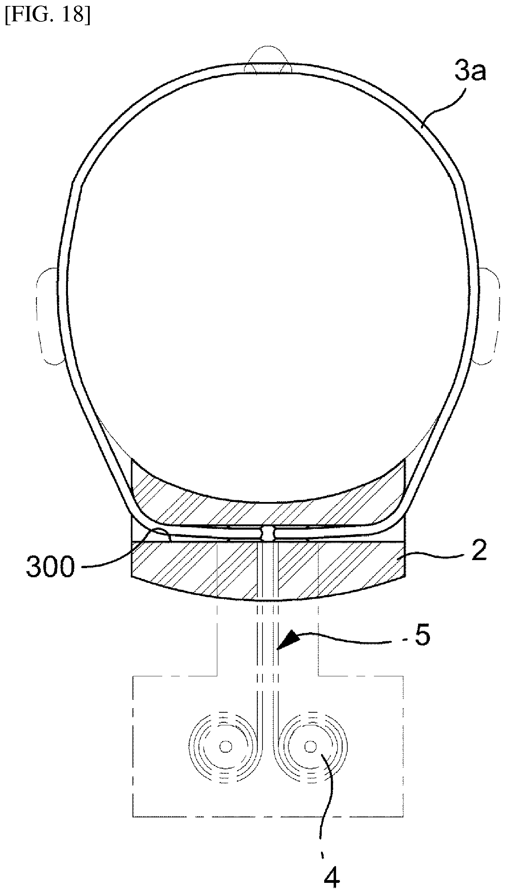

Next, referring to Fig. 18, the head fixing apparatus may include a head holder in the form of a band tied around the head, i.e. a band type head holder 3a, and the occipital region support 2.

In other words, the head holder 3a is a head fixing band that is wrapped around the temporal regions and frontal region of a head to be tightened on the head and presses the back of a head (occipital region) against the occipital region support.

The head fixing band 3a is connected to the left traction portion 5a of the traction member as set forth above at one end thereof, and the head fixing band 3a is connected to the right traction portion 5b of the traction member at the other end thereof. As the traction member 5 is wound on the take-up roll 4 through activation of the motor, the head fixing band 3a retracts to be pressed against the frontal region and temporal regions of the head of the subject, which causes the occipital region to be pressed against the occipital region support 2, thereby allowing the head of a subject to be held in place.

The head holder takes the form of a band made of a flexible material. To components other than the head holder, i.e. components such as the occipital region support, the backrest, and the drive unit, the same descriptions as in the above embodiments may be applied.

In the above embodiment, although the present invention has been described by way of example wherein the head fixing apparatus is configured to be applicable to a typical chair with a back, it should be understood that the head fixing apparatus according to the invention may be configured such that the occipital region support is directly mounted on a chair with a back.

### [Industrial Applicability]

The present invention provides a head fixing apparatus which is used in an image acquisition apparatus including a radiographic apparatus, and serves to hold the head of a subject in an imaging position. The head fixing apparatus may be applied to the field of medical imaging apparatuses.

In addition, the head fixing apparatus according to the invention can minimize or prevent compression of face skin (facial soft tissue) during acquisition of head radiographs, and thus can provide accurate diagnostic data for diagnosis or a procedure requiring interrelation between soft tissues and hard tissues while allowing registration between a facial image and a skull radiograph based on facial soft tissues to be easily and accurately achieved. Further, the head fixing apparatus according to the invention can increase accuracy of registration between a facial image and a skull radiograph, thereby allowing more accurate simulation of plastic surgery/orthodontics.

## Claims

1. A head fixing apparatus for fixing a head of a subject while the subject stands or sits during radiography, comprising:
an occipital region support adapted to support the back of a head; and
a head holder movably provided to the occipital region support and adapted to be tightened on the head to prevent lateral movement of the head **characterized in that** the head fixing apparatus further comprising a drive unit for moving the head holder to be tightened on the head,
wherein the drive unit comprises a traction member pulling the head holder to be tightened on the head, and an actuator applying traction force to the head holder via the traction member.

2. The head fixing apparatus according to claim 1, wherein the head holder comprises:
a left support movably provided to the occipital region support to press a left side of the head; and
a right support movably provided to the occipital region support to press a right side of the head.

3. The head fixing apparatus according to claim 1, wherein the head holder comprises a head fixing band for being wrapped on temporal regions and a frontal region of the head to be tightened on the head and pressing the back of the head against the occipital region support.

4. The head fixing apparatus according to claim 1, wherein the occipital region support is movable up or down for height adjustment.

5. The head fixing apparatus according to claim 1, wherein the occipital region support is supported by a backrest, and the traction member is placed in a traction path formed in the occipital region support and the backrest.

6. The head fixing apparatus according to any one of claims 1 to 4, wherein the occipital region support is supported by a backrest, and the occipital region support is connected to the backrest via a neck support for supporting the back of a neck.

7. The head fixing apparatus according to claim 6, wherein the neck support has a forwardly convex shape such that a cervical spine is curved in the form of the letter "C".

8. The head fixing apparatus according to claim 6, wherein the neck support comprises:
a support frame, and
a cushion provided to the support frame and inflated according to the amount of a fluid supplied thereto to support the back of the neck such that a cervical spine is curved in the form of the letter "C".

9. The head fixing apparatus according to claim 1, wherein the occipital region support is supported by a backrest, and the backrest is provided with a backrest fastening belt fastening the backrest to a backrest support on which the backrest is mounted.

10. The head fixing apparatus according to claim 1, wherein the occipital region support is supported by a backrest, and the backrest is provided with a subject fastening belt for fastening the body of a subject to the backrest.

11. The head fixing apparatus according to claim 1, further comprising: a seat for supporting hips to allow a subject to sit thereon.

12. The head fixing apparatus according to claim 1, wherein the head holder is a bar made of an elastic material or a band made of a flexible material, and the occipital region support is made of an acrylic resin.

## Patentansprüche

1. Ein Kopf-Fixierungsgerät zum Fixieren eines Kopfes einer Testperson, während die Testperson bei einer Radiographie steht oder sitzt, die aufweist:
eine Hinterkopfbereichstütze, die dazu angepasst ist einen Hinterkopf zu stützen; und
eine Kopfstütze, die zu der Hinterkopfbereichstütze beweglich vorgesehen ist, und die dazu angepasst ist, an dem Kopf festgezogen zu werden, um eine seitliche Bewegung des Kopfes zu vermeiden, **gekennzeichnet dadurch, dass** das Kopf-Fixierungsgerät weiterhin eine Antriebseinheit zum Bewegen der an dem Kopf festzuziehenden Kopfstütze aufweist, wobei die Antriebseinheit ein Zugelement aufweist, das die an dem Kopf festzuziehende Kopfstütze zieht, und einen Aktuator, der über das Zugelement eine Zugkraft auf die Kopfstütze ausübt.

2. Das Kopf-Fixierungsgerät gemäß Anspruch 1, wobei die Kopfstütze aufweist:
eine linke Stütze, die zu der Hinterkopfbereichstütze beweglich vorgesehen ist, um eine linke Seite des Kopfes anzudrücken; und
eine rechte Stütze, die zu der Hinterkopfbereichstütze beweglich vorgesehen ist, um eine rechte Seite des Kopfes anzudrücken.

3. Das Kopf-Fixierungsgerät gemäß Anspruch 1, wobei die Kopfstütze ein Kopf-Fixierungsband zum Wickeln um Schläfenbereiche und um einen Vorderkopfbereich des Kopfes, das zum Festziehen an dem Kopf und zum Drücken des Hinterkopfes gegen die Hinterkopfbereichstütze vorgesehen ist, aufweist.

4. Das Kopf-Fixierungsgerät gemäß Anspruch 1, wobei die Hinterkopfbereichstütze zur Höhenverstellung auf und ab bewegbar ist.

5. Das Kopf-Fixierungsgerät gemäß Anspruch 1, wobei die Hinterkopfbereichstütze durch eine Rückenlehne gestützt ist, und wobei das Zugelement in einem Zugpfad angeordnet ist, der in der Hinterkopfbereichstütze und der Rückenlehne ausgebildet ist.

6. Das Kopf-Fixierungsgerät gemäß einem der Ansprüche 1 bis 4, wobei die Hinterkopfbereichstütze durch eine Rückenlehne gestützt ist, und die Hinterkopfbereichstütze mit der Rückenlehne durch eine Nackenstütze zum Stützen eines rückwärtigen Nackens verbunden ist.

7. Das Kopf-Fixierungsgerät gemäß Anspruch 6, wobei die Nackenstütze eine nach vorne konvexe Form aufweist, so dass eine Halswirbelsäule in der Form eines Buchstabens "C" gekrümmt ist.

8. Das Kopf-Fixierungsgerät gemäß Anspruch 6, wobei die Nackenstütze folgendes aufweist:
ein Stützgerüst, und
ein Kissen, das in dem Stützgerüst vorgesehen ist, und das gemäß einer Fluidmenge, die darin bereitgestellt ist, aufgeblasen ist, um den rückwärtigen Nacken zu stützen, so dass eine Halswirbelsäule in der Form des Buchstabens "C" gekrümmt ist.

9. Das Kopf-Fixierungsgerät gemäß Anspruch 6, wobei die Hinterkopfbereichstütze durch eine Rückenlehne gestützt ist, und die Rückenlehne mit einem Rückenlehnen-Befestigungsgurt versehen ist, der die Rückenlehne an einer Rückenlehnenstütze befestigt, auf der die Rückenlehne montiert ist.

10. Das Kopf-Fixierungsgerät gemäß Anspruch 1, wobei die Hinterkopfbereichstütze durch eine Rückenlehne gestützt ist, und wobei die Rückenlehne mit einem Testpersonen-Befestigungsgurt versehen ist, um den Körper einer Testperson an der Rückenlehne zu befestigen.

11. Das Kopf-Fixierungsgerät gemäß Anspruch 1, das weiter aufweist: einen Sitz zum Stützen von Hüften, um es einer Testperson zu ermöglichen, darauf zu sitzen.

12. Das Kopf-Fixierungsgerät gemäß Anspruch 1, wobei die Kopfstütze eine aus einem elastischen Material hergestellte Stange oder ein aus einem flexiblen Material hergestelltes Band ist, und wobei die Hinterkopfbereichstütze aus einem Acrylharz hergestellt ist.

## Revendications

1. Un dispositif de fixation de tête pour fixer une tête d'un sujet quand le sujet s'assoit ou se tient debout pendant une radiographie, comprenant :
un support de région occipitale adapté à supporter l'arrière d'une tête; et
un porteur de tête qui est pourvu de façon mobile par rapport au support de région occipitale et qui est adapté pour être serré sur la tête pour éviter un mouvement latéral de la tête, **caractérisé en ce que**
le dispositif de fixation de tête comprend une unité d'entraînement pour mouvoir le porteur de tête afin d'être serré sur la tête,
dans lequel l'unité d'entraînement comprend un élément de traction qui tire le porteur de tête afin d'être serré sur la tête, et un actuateur qui applique une force de traction au porteur de tête par l' élément de traction.

2. Le dispositif de fixation de tête selon revendication 1, dans lequel le porteur de tête comprend :
un support gauche qui est pourvu de façon mobile par rapport au support de région occipitale pour appuyer un côté gauche de la tête; et
un support droit qui est pourvu de façon mobile par rapport au support de région occipitale pour appuyer un côté droit de la tête.

3. Le dispositif de fixation de tête selon revendication 1, dans lequel le support de tête comprend un ruban de fixation pour être enroulé sur des régions temporales et sur une région frontale de la tête afin d'être serré sur la tête et afin d'appuyer l'arrière de la tête contre le support de région occipitale.

4. Le dispositif de fixation de tête selon revendication 1, dans lequel le support de région occipital est déplaçable vers le haut et vers le bas pour l'ajustement en hauteur.

5. Le dispositif de fixation de tête selon revendication 1, dans lequel le support de région occipitale est supporté par un appui-dos, et l'élément de traction est placé dans un chemin de traction formé dans le support de région occipitale et l'appui-dos.

6. Le dispositif de fixation de tête selon l'une quelconque des revendications 1 à 4, dans lequel le support de région occipitale est supporté par un appuie-dos, et le support de région occipitale est connecté au moyen de l'appui-dos par un support de nuque pour supporter l'arrière d'une nuque.

7. Le dispositif de fixation de tête selon revendication 6, dans lequel le support de nuque a une forme convexe vers l'avant de sorte qu'une colonne cervicale est courbée en forme d'une lettre "C".

8. Le dispositif de fixation de tête selon revendication 6, dans lequel le support de nuque comprend :
un cadre de support, et
un coussin pourvu au cadre de support et gonflé selon une quantité de fluide appliquée à celui-ci pour supporter l'arrière d'une nuque de sorte qu'une colonne cervicale est courbée dans la forme d'une lettre "C".

9. Le dispositif de fixation de tête selon revendication 1, dans lequel le support de région occipitale est supporté par un appui-dos, et l'appui-dos est pourvu d'une ceinture de fixation qui fixe l'appui-dos à un support d'appui-dos sur lequel l'appui-dos est monté.

10. Le dispositif de fixation de tête selon revendication 1, dans lequel le support de région occipital est supporté par un appui-dos, et l'appui-dos est pourvu d'une ceinture de fixation de sujet pour fixer le corps d'un sujet à l'appui-dos.

11. Le dispositif de fixation de tête selon revendication 1, comprenant en outre : un siège pour supporter des hanches afin de permettre un sujet de s'asseoir sur celui-ci.

12. Le dispositif de fixation de tête selon revendication 1, dans lequel le support de tête est une barre faite d'un matériau élastique ou un ruban fait d'un matériau flexible, et le support de région occipital est fait d'une résine acrylique.
